(19) Europäisches Patentamt
European Patent Office
Office européen des brevets

(11) **EP 4 556 588 A1**

(12) **EUROPEAN PATENT APPLICATION**
published in accordance with Art. 153(4) EPC

(43) Date of publication:
**21.05.2025 Bulletin 2025/21**

(21) Application number: **24780101.2**

(22) Date of filing: **25.03.2024**

(51) International Patent Classification (IPC):
**C22C 19/03** (2006.01)    **C22F 1/00** (2006.01)
**C22F 1/10** (2006.01)

(52) Cooperative Patent Classification (CPC):
**C22C 19/03; C22F 1/00; C22F 1/10**

(86) International application number:
**PCT/JP2024/011547**

(87) International publication number:
**WO 2024/203983 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **27.03.2023 JP 2023049844**

(71) Applicants:
• **Furukawa Techno Material Co., Ltd.
Kanagawa 254-0016 (JP)**
• **FURUKAWA ELECTRIC CO., LTD.
Tokyo 100-8322 (JP)**

(72) Inventors:
• **FUJII, Misato**
**Hiratsuka-shi, Kanagawa 254-0016 (JP)**
• **UEOKA, Tsuyoshi**
**Hiratsuka-shi, Kanagawa 254-0016 (JP)**
• **NAKAKUMA, Syogo**
**Hiratsuka-shi, Kanagawa 254-0016 (JP)**
• **TADA, Hidefumi**
**Hiratsuka-shi, Kanagawa 254-0016 (JP)**

(74) Representative: **Papula Oy**
**P.O. Box 981
00101 Helsinki (FI)**

(54) **TUBE MATERIAL, METHOD FOR MANUFACTURING TUBE MATERIAL, STENT, GUIDE WIRE, AND PRESSURE GUIDE WIRE**

(57)    Provided are a tubing material which can be industrially manufactured by a simple method, consisting of a Ni-Ti-based alloy material, having improved roughness of the inner wall surface, and superior in durability against rotary bending, a method for producing this, as well as a stent, a guide wire, and a pressure guide wire made using the tubing material. The tubing material consists of a Ni-Ti-based alloy, in which an arithmetic mean height Sa of an inner wall surface is 0.08 μm or more and 0.55 μm or less.

FIG .1

Processed by Luminess, 75001 PARIS (FR)

**Description**

TECHNICAL FIELD

The present disclosure relates to a tubing material, a method for producing the tubing material, a stent, a guide wire and a pressure guide wire.

BACKGROUND ART

Ni-Ti-based alloy materials are superior in corrosion resistance and abrasion resistance. In addition, Ni-Ti-based alloy materials are known to exhibit shape memory property and superelastic property, and have special properties which enable to withstand deformation of 5% or more for which the deformation would remain in ordinary metals, and to return to the original shapes without producing plastic deformation. Further, it has been found that the Ni-Ti-based alloy materials exhibit resistance to repetitive deformation in a state in which a certain strain is applied.

In recent years, accompanying the development of technology for machining Ni-Ti-based alloy materials into ultrafine wires and capillaries, the application of Ni-Ti-based alloy materials has been expanding to medical devices such as stents, artificial heart valves, and guide wires used for inserting a stent or catheter into the body.

Among medical devices, stents and artificial heart valves are called indwelling type devices, and due to being subjected to the repetitive load of contraction and expansion accompanying the pulsatory motion of blood vessels or the like in a living body, they are devices having many advanced required characteristics, for which excellent fatigue durability over a long period of time of 10 years or more is desired.

On the other hand, applications such as disposable devices (hereinafter, also referred to as disposable) different from the above-described medical devices such as the indwelling type devices are also greatly expanding. Disposables have many methods of use such that target and remove a local blockage in a narrow-diameter blood vessel. In order to make a device pass through the blood vessel to reach the affected area, the device needs to be rotated and bent. In addition, there are numerous methods for further passing a wire or other device through the inside of a tubing material after the device has reached the affected area.

In view of such background, fracture resistance against rotary bending is particularly demanded in tubing materials. In addition, in applications in which another device is passed through the inside of the tubing material, since an improvement is demanded in operability so that the other device is smoothly passed through the inside of the tubing material, the state of the inner wall surface of the tubing material is important.

In addition, conventionally, various considerations have been made for tubing materials consisting of Ni-Ti-based alloys.

For example, Patent Documents 1 and 2 provide inventions from the viewpoint of a roughness improvement of the inner wall surface of the tubing material being important. Furthermore, based on these technologies, Non-patent Documents 1 and 2 achieve the obtaining of a tubing material of 4 mm outer diameter or less having favorable roughness of the inner wall surface.

However, when summarizing Non-patent Documents 1 and 2, in order to obtain capillaries of 4 mm outer diameter or less having good inner wall surface state, a fixed plug drawing is performed at a stage with outer diameter of 4 mm or more, and a mandrel drawing is performed at a stage with outer diameter of 4 mm or less. In this way, due to requiring such joint use of different steps, it is difficult to industrial produce long-thin diameter tubing materials.

In addition, in Patent Document 1 which is using a core made of a shape memory alloy, a tubing material having an inner wall surface of 20 s or less (arithmetic mean roughness Ra is about 5 μm) is obtained, and in Patent Document 2 in which float plug drawing known as a manufacturing method of a long tubing material is performed, a tubing material having an inner wall surface of 5 s or less (arithmetic mean roughness Ra is about 1.25 μm) is obtained. However, in order to apply the tubing materials of Patent Documents 1 and 2 to the above applications, the roughness of the inner wall surface of the tubing material is large, and thus there is room for further improvement.

Herein, an outline of the prior art related to methods for producing tubing materials consisting of shape-memory alloys will be described. Patent Documents 3 and 4 describe production methods of integral processing by inserting a core consisting of a shape-memory alloy into a metal tube. By charging a cold worked core into a metal tube and then heating, the gap between the metal tube and the core becomes the smallest to form a composite material in which the metal tube and the core are integrated. Processing to the target dimensions of the metal tube (tubing material) in the state of a composite material becomes possible, and thus the manufacture of a tubing material with good dimensional precision having controlled the outer diameter and the wall thickness becomes industrially possible. Thereafter, it is necessary to remove the core from the tubing material to obtain a tubing material processed to the target dimensions. With Patent Documents 3 and 4, it is necessary to heat the entire composite material after processing to 750 to 825°C, or to perform electrical heating on only the core, and the distortion of the core is eliminated by heating, and subsequently, the core is pulled out by a drawbench, whereby stretching the core to reduce the diameter of the core, and thus the removal of the core from the tubing material is said to be possible.

In addition, Patent Documents 5 and 6 describe method of forming a composite material by inserting a core made of mild steel, carbon steel, stainless steel, brass, or the like into a tubing material consisting of a shape-memory alloy, and then integral processing the composite material. In Patent Documents 5 and 6, as a method for removing the core, when heating the composite material after processing, only the seamless tubing material greatly swells by thermal expansion, and a gap (hereinafter, also referred to as clearance) is formed between the tubing material and the core. By forming this clearance, it becomes possible to remove the core from the tubing material.

Although Patent Documents 3 to 6 are effective techniques for manufacturing tubing material, there is still room for improvement in the manufacture of small diameter tubing materials of 4 mm or less. In particular, in the step of removing the core, in particular, in the case of being a small diameter tubing material, the precision of the clearance and whether or not the tubing material and the core are reliably detached from each other are industrially important points. For example, when the core is extracted from the tubing material in a state where the clearance is insufficient or the detachment between the tubing material and the core is insufficient, a problem in that streaks are formed along the tubing material longitudinal direction in the inner wall surface of the tubing material has been confirmed.

In addition, all of the above-described technologies are technologies which have been developed before 2004. Therefore, there is still room for improvement in the conditions that can be adapted to ASTM F2063-18(Standard Specification for Wrought Nickel-Titanium Shape Memory Alloys for Medical Device and Surgical Implants), which is defined for medical Ni-Ti-based alloys. When used in a medical device, the above standard is required not only for indwelling type devices, but also for disposable type devices. In particular, the allowable levels for the carbon concentration and the oxygen concentration are revised to a maximum of 0.04% by mass respectively, and the range to which the carbon concentration and the oxygen concentration are controlled becomes even narrower than that before the above standard. Therefore, technological development is needed for the manufacturing of small diameter tubing material, while optimizing the manufacturing conditions for performing the control of these element concentrations.

Further, with the conventional technology, when evaluating the roughness of the inner wall surface, since the arithmetic mean roughness Ra measured linearly along the longitudinal direction of the tubing material is evaluated, the influence of the roughness cannot be quantitatively determined with respect to scratches formed along the longitudinal direction of the tubing material. Therefore, there is room for improvement also in the technology for evaluating the roughness of the inner wall surface of the tubing material.

Based on the above, a tubing material made of a Ni-Ti-based alloy having improved roughness of the inner wall surface has been demanded which can be manufactured by an industrially easy method.

Citation List

Patent Document

Patent Document 1: Japanese Unexamined Patent Application, Publication No. H10-17963
Patent Document 2: Japanese Unexamined Patent Application, Publication No. H11-61301
Patent Document 3: US Patent No. 5709021
Patent Document 4: US Patent No. 6799357
Patent Document 5: Japanese Patent No. 3443206
Patent Document 6: Japanese Patent No. 2133478

Non-Patent Document

Non-Patent Document 1: J. Materials processing Technology,118(2001),p.251-255
Non-Patent Document 2: J. Materials processing Technology,153-154(2004),p.145-150

DISCLOSURE OF THE INVENTION

Problems to be Solved by the Invention

An object of the present disclosure is to provide a tubing material which can be industrially manufactured by a simple method, consisting of a Ni-Ti-based alloy material, having improved roughness of the inner wall surface, and superior in durability against rotary bending, a method for producing this, as well as a stent, a guide wire, and a pressure guide wire made using the tubing material.

Means for Solving the Problems

[1] A tubing material consists of a Ni-Ti-based alloy, in which an arithmetic mean height Sa of an inner wall surface is 0.08 $\mu$m or more and 0.55 $\mu$m or less.

[2] In the tubing material as described in [1], the tubing material contains 54.5% by mass or more and 57.0% by mass or less of Ni, and a balance consists of Ti and inevitable impurities.

[3] In the tubing material as described in [1] or [2], a ratio (outer diameter/inner diameter) of an outer diameter to an inner diameter of the tubing material is 1.01 or more and 5.00 or less.

[4] In the tubing material as described in any one of [1] to [3], an outer diameter of the tubing material is 0.15 mm or more and 4.00 mm or less, and an inner diameter of the tubing material is 0.10 mm or more and 3.95 mm or less.

[5] In the tubing material as described in any one of [1] to [4], the tubing material has superelastic property at 37°C.

[6] A stent formed from the tubing material as described in any one of [1] to [5].

[7] A guide wire formed from the tubing material as described in any one of [1] to [5].

[8] A pressure guide wire formed from the tubing material as described in any one of [1] to [5].

[9] A method for producing the tubing material as described in any one of [1] to [5], the method including: a melting and casting step for a Ni-Ti-based alloy material (Step 1); a hot working step (Step 2); a drawing step of drawing a composite material obtained by inserting a core into the tubular Ni-Ti-based alloy hot-worked material obtained in the hot working step (Step 2) to obtain a drawn composite material in which a tubular Ni-Ti-based alloy drawn material and a drawn core material extending inside of the Ni-Ti-based alloy drawn material are integrated (Step 3); and a core removing step of heating the Ni-Ti-based alloy drawn material at 150°C or higher and 800°C or lower, while applying, to the drawn core material in the drawn composite material, a tensile load of a magnitude at which the Ni-Ti-based alloy drawn material in the drawn composite material does not detach from the drawn core material (Step 4).

[10] In the method for producing the tubing material as described in [9], in which, in the core removing step (Step 4), the Ni-Ti-based alloy drawn material is heated at 150°C or higher and 800°C or lower, while applying a tensile load of 4 MPa or more and 300 MPa or less to the drawn core material in the drawn composite material.

[11] The method for producing the tubing material as described in [9] or [10] further includes a superelastic property imparting heat treating step of conducting a superelastic property imparting heating on the tubing material (Step 5) performed after the core removing step (Step 4).

Effects of the Invention

According to the present disclosure, it is possible to provide a tubing material which can be industrially manufactured by a simple method, consisting of a Ni-Ti-based alloy material, having improved roughness of the inner wall surface, and superior in durability against rotary bending, a method for producing this, as well as a stent, a guide wire, and a pressure guide wire made using the tubing material.

BRIEF DESCRIPTION OF THE DRAWINGS

FIG. 1 is a perspective view showing an example of a tubing material according to an embodiment.

FIG. 2 is a cross-sectional view showing an example of a tensile load applying step (Step 4-1).

FIG. 3 is a cross-sectional view showing another example of a tensile load applying step (Step 4-1).

FIG. 4 is a cross-sectional view showing a state after performing an annealing step (Step 4-2) on a drawn composite material with the configuration shown in FIG. 2.

FIG. 5 is a cross-sectional view showing a state after performing an annealing step (Step 4-2) on a drawn composite material with the configuration shown in FIG. 3.

FIG. 6 is a cross-sectional view showing an example of a core diameter-reducing step (Step 4-3).

FIG. 7 is a cross-sectional view showing an example of a core extracting step (Step S4-4).

FIG. 8 is a schematic drawing showing a dual-drive type rotary bending fatigue tester used in Examples and Comparative Examples.

FIG. 9 provides the results of observing an inner wall surface of a tubing material produced in Example 1 by a 3D measurement laser microscope.

FIG. 10 provides the results of observing an inner wall surface of a tubing material produced in Example 10 by a 3D measurement laser microscope.

FIG. 11 provides the results of observing an inner wall surface of a tubing material produced in Comparative Example 6 by a 3D measurement laser microscope.

PREFERRED MODE FOR CARRYING OUT THE INVENTION

Hereinafter, the present invention will be described in detail based on embodiments.

As a result of intensive study, the present inventors have found a production method capable of improving the roughness of the inner wall surface of a tubing material without performing the usual steps such as float plug pulling, fixed plug pulling, and mandrel pulling, which have been known for improving the roughness of the inner wall surface of a tubing material, and a step of physically or chemically treating the inner wall surface of a tubing material by polishing or the like. Furthermore, the present inventors have found that, by improving the roughness of the inner wall surface of the tubing material, a superior characteristic in durability against rotary bending can be obtained. Based on such knowledge, the present disclosure arrived at completion.

The tubing material of the embodiment consists of a Ni-Ti-based alloy, and the arithmetic mean height Sa of the inner wall surface is 0.08 $\mu$m or more and 0.55 $\mu$m or less.

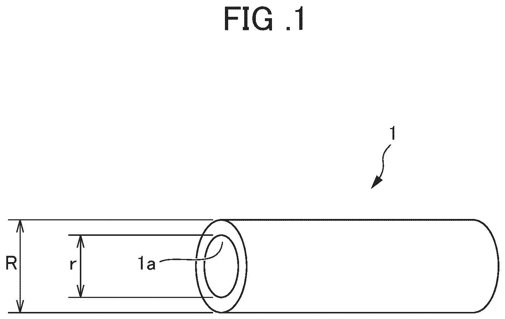

FIG. 1 is a perspective view showing an example of a tubing material according to an embodiment. The tubing material 1 shown in FIG. 1 is made of a Ni-Ti-based alloy, and the arithmetic mean height Sa of the inner wall surface 1a is 0.08 $\mu$m or more and 0.55 $\mu$m or less.

If the upper limit value for the arithmetic mean height Sa of the inner wall surface 1a of the tubing material 1 is 0.55 $\mu$m or less, the roughness of the inner wall surface 1a is improved, and the tubing material 1 can have superior durability against rotary bending. From such a viewpoint, the arithmetic mean height Sa of the inner wall surface 1a of the tubing material 1 is 0.55 $\mu$m or less, preferably 0.45 $\mu$m or less, and is preferably as small as possible. In addition, the lower limit value for the arithmetic mean height Sa of the inner wall surface 1a is 0.08 $\mu$m or more, from the viewpoint of ease of processing.

The arithmetic mean height Sa is a parameter obtained by expanding, in a plane, the arithmetic mean roughness Ra of the linear measurement used in the general roughness evaluation. The arithmetic mean height Sa of the inner wall surface 1a can be obtained by measuring the inner wall surface 1a of the tubing material 1 using a 3D measurement laser microscope, after vertically dividing the tubing material 1 in the longitudinal direction.

The tubing material 1 consisting of a Ni-Ti-based alloy is mainly composed of a matrix phase (matrix) which is not shown, and a plurality of non-metallic inclusions (not shown) existing in the matrix phase. In addition, the tubing material 1 preferably has superelastic property.

In addition, in order to improve the processability, it is preferable for the tubing material 1 to contain 54.5% by mass or more and 57.0% by mass or less of Ni (nickel), and the balance to consist of Ti (titanium) and inevitable impurities. Among such alloy compositions, since the tubing material 1 will conform to the conditions of ASTM F2063-18 defined for medical Ni-Ti-based alloys when the content ratio of C (carbon) contained in the tubing material 1 is 0.04% by mass or less and the content ratio of O (oxygen) contained in the tubing material 1 is 0.04% by mass or less, the tubing material 1 can be applied to medical applications.

Ni is an element required for exhibiting superelastic property and shape memory property in a Ni-Ti-based alloy. When the Ni content is outside the range of 54.5% by mass or more and 57.0% by mass or less, the processing of a tube shape will not be easy. Therefore, in case of emphasizing processability, the lower limit value for the Ni content is preferably 54.5% by mass or more, more preferably 55.6% by mass or more, and the upper limit value thereof is preferably 57.0% by mass or less, and more preferably 56.7% by mass or less.

C is an element that forms non-metallic inclusions. When the C content becomes abundant, the number of non-metallic inclusions present in the matrix phase increases, and the occupation ratio of the non-metallic inclusions in the tubing material 1 becomes higher, so that premature fracture (fracture at a small number of rotations) of the tubing material 1 easily occurs. Therefore, a smaller C content is more preferable. In particular, according to the definition in ASTM F2063-18, the C content is preferably 0.04% by mass or less.

O is an element that forms non-metallic inclusions. When the O content becomes abundant, the particle diameter of the non-metallic inclusions is increased, so that premature fracture of the tubing material 1 easily occurs. Therefore, a smaller O content is more preferable. In particular, according to the definition in ASTM 2063-18, the O content is preferably 0.04% by mass or less.

Furthermore, in order to adjust superelastic property and shape memory property, the tubing material 1 may contain at least one element selected from the group consisting of Cu (copper), Ta (tantalum), Zr (zirconium), Nb (niobium), V (vanadium), Mo (molybdenum), Cr (chromium), Fe (iron), and Co (cobalt) in a total amount of more than 0.00% by mass and 0.05% by mass or less. These elements are elements included in the tubing material 1 as necessary in order to conduct adjustment of superelastic property and shape memory property. When the concentration of each of the elements is 0.05% by mass or less, the elements will not affect the arithmetic mean height Sa of the inner wall surface 1a of the tubing material 1.

The balance other than the above-mentioned components is Ti and inevitable impurities. The inevitable impurities indicate impurities at a content level that inevitably mixes in the manufacturing process. Since the inevitable impurities may affect the properties of the tubing material depending on the content of the inevitable impurities, a smaller content of the inevitable impurities is more preferable. For example, the content of N (nitrogen), which is an inevitable impurity, is preferably 0.005% by mass or less.

In addition, the matrix phase constituting the tubing material 1 is an austenitic phase consisting of a Ni-Ti-based alloy, and has a B2-type crystal structure with a CsCl-type body-centered cubic lattice structure.

As the non-metallic inclusions present in the matrix phase of the tubing material 1 consisting of the Ni-Ti-based alloy, TiC-based inclusions mainly containing carbides such as TiC, and non-TiC-based inclusions mainly containing oxynitrides such as $Ti_4Ni_2O_x$ are mainly mixed therein. When the amount of the non-metallic inclusions present in the matrix phase becomes abundant, premature fracture starting from the non-metallic inclusions is likely to occur. Among the non-metallic inclusions present in the matrix phase, the non-metallic inclusions that are particularly likely to be the starting point of such premature fracture are non-TiC-based inclusions, and above all, are $Ti_4Ni_2O_x$.

In addition, the ratio (outer diameter R/inner diameter r) of the outer diameter R to the inner diameter r of the tubing material 1 is preferably 1.01 or more and 5.00 or less, and more preferably 1.01 or more and 2.50 or less. When the ratio (outer diameter R/inner diameter r) of the tubing material 1 is within the above range, the durability against rotary bending will be superior even if the tubing material 1 has a small diameter, and furthermore, the tubing material 1 can be suitably used in a medical device.

In addition, it is preferable for the outer diameter R of the tubing material 1 to be 0.15 mm or more and 4.00 mm or less, and for the inner diameter r of the tubing material 1 to be 0.10 mm or more and 3.95 mm or less. When the outer diameter R and the inner diameter r of the tubing material 1 are within the above ranges, the durability against rotary bending will be superior even if the tubing material 1 has a small diameter, and further, the tubing material 1 can be suitably used in a medical device.

In addition, when the tubing material 1 has superelastic property at 37°C, since the tubing material 1 can exhibit superelastic property in a living body, the tubing material 1 can be suitably used for medical devices.

Next, a method of producing the tubing material according to the embodiment will be described.

A method of producing a tubing material according to an embodiment includes a melting and casting step (Step 1) for a Ni-Ti-based alloy material; a hot working step (Step 2); a drawing step (Step 3) of drawing a composite material obtained by inserting a core into the tubular Ni-Ti-based alloy hot-worked material obtained in the hot working step (Step 2) to obtain a drawn composite material in which a tubular Ni-Ti-based alloy drawn material and a drawn core material extending inside of the Ni-Ti-based alloy drawn material are integrated; a core removing step (Step 4) of heating the Ni-Ti-based alloy drawn material at 150°C or higher and 800°C or lower, while applying, to the drawn core material in the drawn composite material, a tensile load of a magnitude at which the Ni-Ti-based alloy drawn material in the drawn composite material does not detach from the drawn core material. The tubing material produced by this production method is the tubing material 1 according to the above embodiment.

In the melting and casting step (Step 1) for the Ni-Ti-based alloy material, the Ni-Ti-based alloy material is melted, and then cast to obtain a Ni-Ti-based alloy ingot. Preferably, both the carbon concentration ([C]) and the oxygen concentration ([O]) in the Ni-Ti-based alloy ingot are adjusted to 0.04% by mass or less.

In addition, when the ([C]/[O]) ratio is 0.5 or more in Step 1, since the generation of $Ti_4Ni_2O_x$ in the ingot obtained at Step 1 is suppressed, the fatigue durability required for in particular an indwelling device can be improved.

As a method of melting the Ni-Ti-based alloy material, melting of the Ni-Ti-based alloy material is performed in a vacuum atmosphere or an inert gas atmosphere such as Ar gas by a high frequency melting method.

The carbon concentration (% by mass) is adjusted by weighing the material so that the total amount of carbon involving the amount of carbon eluted from the used crucible becomes a predetermined amount. The oxygen concentration (% by mass) is adjusted by selecting the grade of the titanium metal and weighing so that the amount of oxygen becomes a predetermined amount on the basis of the fact that the amount of oxygen contained in the titanium metal differs depending on the grade of the titanium metal that is the raw material.

The carbon concentration and the oxygen concentration can be measured using a conventionally known carbon concentration analyzer and oxygen concentration analyzer, respectively.

The hot working step (Step 2) performed after Step 1 includes a forging step (Step 2-1), and may further include an extruding step (Step 2-2).

In the forging step (Step 2-1), the Ni-Ti-based alloy ingot obtained in Step 1 is forged to obtain a Ni-Ti-based alloy hot-worked material. The heating temperature during forging of the Ni-Ti-based alloy ingot is preferably 500°C or higher and 950°C or lower, and more preferably 600°C or higher and 800°C or lower. In Step 2-1, press forging or air hammer forging can be employed.

Subsequently, the Ni-Ti-based alloy hot-worked material is drilled with a gun drill, and then finish processed with a lathe to obtain a seamless tubular Ni-Ti-based alloy hot-worked material.

In addition, the extruding step (Step 2-2) may be performed after the forging step (Step 2-1). In the extruding step (Step 2-2), the Ni-Ti-based alloy hot-worked material obtained in Step 2-1 is extruded, thereby obtaining a seamless tubular Ni-Ti-based alloy hot-worked material. The heating temperature during extrusion of the Ni-Ti-based alloy hot-worked material is preferably 500°C or higher and 950°C or lower, and more preferably 600°C or higher and 800°C or lower. It should be noted that, since Patent Document 5 has the intention of securing a high cross section reduction rate from the viewpoint of emphasizing production efficiency, the optimum processing temperature during extrusion is 800 to 1000°C. On the other hand, in the present embodiment, the heating temperature range during extrusion is different from that of Patent Document 5 for the purpose of maintaining both the carbon concentration ([C]) and the oxygen concentration ([O]) controlled in Step 1 to 0.04% by mass or less respectively, from the viewpoint of the control of inclusions (metallic structures) being important for the tubing material to be used for a medical device.

In the drawing step (Step 3) performed after Step 2, drawing is performed on the composite material obtained by inserting the core into the tubular Ni-Ti-based alloy hot-worked material obtained in Step 2.

First, by inserting the core into the tubular Ni-Ti-based alloy hot-worked material obtained in Step 2, a composite material is produced in which the core is inserted inside of the tubular Ni-Ti-based alloy hot-worked material. At the time of inserting the core into the tubular hot-worked material, although it is necessary to take care so that an insertion flaw along the longitudinal direction of the hot-worked material is not formed on the inner wall surface of the hot-worked material, the degree of difficulty differs depending on the inner diameter dimension of the hot-worked material. Therefore, in the case where insertion of the core is difficult, a lubricant may be applied to the inner wall surface of the hot-worked material or the outer wall surface of the core. The lubricant is preferably a phosphate film, graphite, molybdenum disulfide, or boron nitride.

For the tubular hot-worked material before the core is inserted, the outer diameter is preferably 0.75 mm or more and 51.00 mm or less, more preferably 2.50 mm or more and 10.00 mm or less, and the inner diameter is preferably 0.50 mm or more and 9.00 mm or less. The ratio of the inner diameter to the outer diameter of the hot-worked material is preferably 1.01 or more and 5.00 or less, more preferably 1.01 or more and 2.50 or less, and still more preferably 1.15 or more and 2.00 or less.

Here, when the dimensional difference between the outer diameter of the core and the inner diameter of the hot-worked material before inserting the core into the hot-worked material is set to 2% or more and 25% or less, since the clearance at the time of inserting the core can be sufficiently secured, simple assembly of the composite material becomes possible. In addition, when the deformation resistance of the core is set to the same level as the deformation resistance of the hot-worked material, buckling of the inner wall surface of the hot-worked material can be suppressed, and the processing from the hot-worked material to the small diameter tubing material can be performed in a favorable state. In this case, for the purpose of adjusting the deformation resistance of the core and the hot-worked material, the composite material may be subjected to the warm working described later.

The core before insertion into the hot-worked material is preferably cold worked. Here, the present inventors have found that, for the arithmetic mean height Sa of the inner wall surface of the tubing material, the roughness state of the outer wall surface of the core before insertion is important. Therefore, the arithmetic mean height Sa of the outer wall surface of the core is preferably 0.03 μm or more and 0.30 μm or less, and more preferably 0.03 μm or more and 0.20 μm or less, when measured based on the description in "[3] Arithmetic mean height Sa of the outer wall surface of the core" described later. Regarding the annealing condition of the core, although annealing may be partially performed in an air atmosphere, the finishing is preferably performed in an inert atmosphere.

Examples of the material of the core include SUS304, SUJ2, SS400, SCM415, SUS316, SUS309S, SUS310S and Ni(54.5 to 56.0% by mass)-Ti (+ inevitable impurities); however, it is not to be limited thereto.

Subsequently, drawing is performed on the composite material. By performing drawing, a drawn composite material in which the composite material has been drawn is obtained. The drawn composite material is formed by integrating a tubular Ni-Ti-based alloy drawn material in which the tubular Ni-Ti-based alloy hot-worked material has been drawn and a drawn core material which extends inside of the Ni-Ti-based alloy drawn material and in which the core has been drawn.

The drawing process is cold working or warm working. The cold working performs drawing without putting the composite material into a temperature adjusted furnace. The warm working performs drawing the composite material while controlling the temperature of the composite material, and is performed for the purpose of adjusting the deformation resistance of the core and the hot-worked material. The temperature of the warm working is preferably 100°C or higher and 800°C or lower as a set temperature inside the furnace. The heating atmosphere may be an air atmosphere due to not affecting the state of the inner wall surface of the tubing material; however, it is preferably an inert atmosphere for the purpose of maintaining the state of the outer wall surface of the tubing material.

The processing rate of Step 3 is at least 10% or more and less than 60%.

In addition, multiple elongating through a die having a continuously reducing diameter may be applied to the composite material.

By Step 3, the drawn composite material having an outer diameter of 0.15 mm or more and 4.00 mm or less is obtained.

In the drawing step (Step 3), drawing and annealing may be repeatedly conducted on the composite material. In order to restore by removing the strain generated in the composite material (tubular Ni-Ti-based alloy processed material) or the drawn composite material (tubular Ni-Ti-based alloy drawn material) at the drawing process through annealing, annealing is performed during the drawing process or after the drawing process.

The annealing temperature in Step 3 is preferably 500°C or higher and 800°C or lower, and more preferably 600°C or higher and 800°C or lower. The heating atmosphere for the annealing may be an air atmosphere due to not affecting the state of the inner wall surface of the tubing material; however, it is preferably an inert atmosphere for the purpose of maintaining the state of the outer wall surface of the tubing material.

Drawing and annealing of the composite material are repeated until reaching the above dimensions.

In the core removing step (Step 4) conducted after Step 3, only the drawn core material is removed from the drawn composite material obtained in Step 3 to obtain a tubing material. The core removing step (Step 4) includes a tensile load applying step (Step 4-1), an annealing step (Step 4-2), a core diameter-reducing step (Step 4-3), and a core extracting step (Step 4-4). The core diameter-reducing step (Step 4-3) is an optional step.

In the tensile load applying step (Step 4-1), a tensile load of a magnitude at which the tubular Ni-Ti-based alloy drawn material of the drawn composite material does not detach from the drawn core material is applied to the drawn core material of the drawn composite material. The tensile load applied to the drawn core material is preferably 4 MPa or more and 300 MPa or less, and more preferably 20 MPa or more and 150 MPa or less.

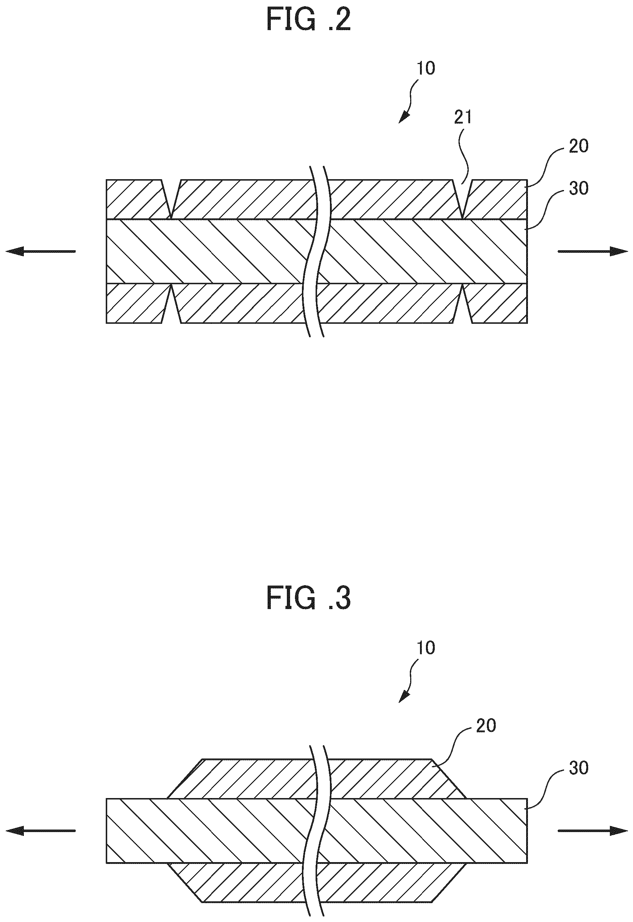

FIG. 2 is a cross-sectional view showing an example of the tensile load applying step (Step 4-1), and FIG. 3 is a cross-sectional view showing another example of the tensile load applying step (Step 4-1). As shown in FIG. 2, score lines 21 may be formed along the circumferential direction at both end portions of the tubular Ni-Ti-based alloy drawn material 20 constituting the drawn composite material 10. The score lines 21 penetrate from the outer wall surface to the inner wall surface of the Ni-Ti-based alloy drawn material 20, for example. With such a configuration, a tensile load is applied to the drawn core material 30, while grasping portions of the drawn composite material 10 closer to the end side than the score line 21. In addition, as shown in FIG. 3, both end portions of the Ni-Ti-based alloy drawn material 20 may be cut to expose the drawn core material 30 at both end portions of the drawn composite material 10. With such a configuration, a tensile load is applied to the drawn core material 30, while grasping the drawn core material 30 exposed at both end portions of the drawn composite material 10.

When the tensile load applied to the drawn core material 30 of the drawn composite material 10 is greater than the above range, that is, when a tensile load of a magnitude at which the Ni-Ti-based alloy drawn material 20 of the drawn composite material 10 is detached from the drawn core material 30 is applied, since plastic deformation occurs in the Ni-Ti-based alloy drawn material 20, the material characteristics of the tubing material 1 including the arithmetic mean height Sa deteriorate. As a result, the arithmetic mean height Sa of the inner wall surface 1a of the tubing material 1 becomes more than 0.55 $\mu$m.

In the annealing step (Step 4-2) performed after the tensile load applying step (Step 4-1), the Ni-Ti-based alloy drawn material 20 is heated in a state where the tensile load with the above magnitude is applied to the drawn core material 30 in the drawn composite material 10 in the tensile load applying step (Step 4-1). The heating temperature is 150°C or higher and 800°C or lower, and is preferably 450°C or higher and 700°C or lower.

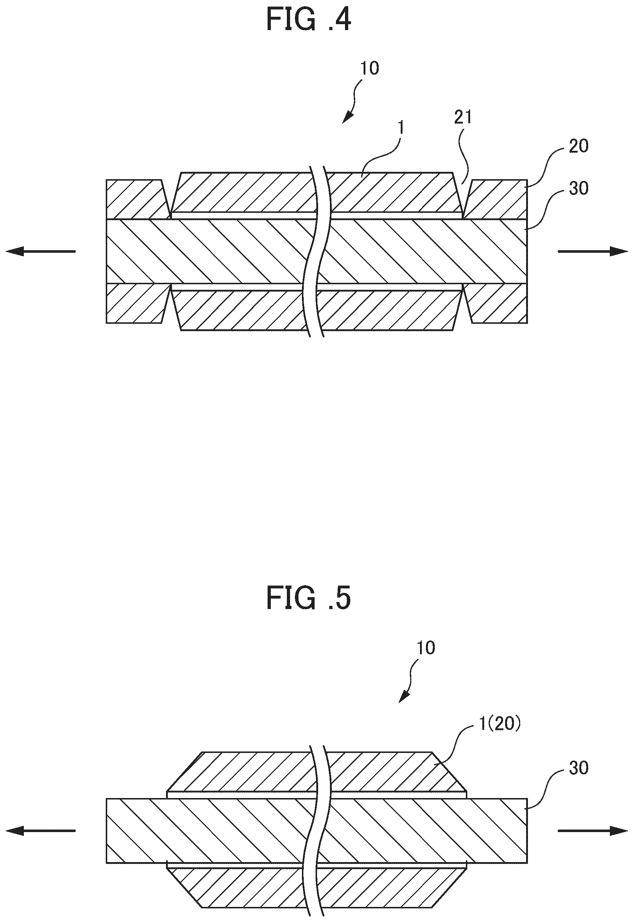

FIG. 4 is a cross-sectional view showing a state after the annealing step (Step 4-2) is performed on the drawn composite material 10 having the configuration shown in FIG. 2, and FIG. 5 is a cross-sectional view showing a state after the annealing step (Step 4-2) is performed on the drawn composite material 10 having the configuration shown in FIG. 3. As shown in FIGS. 4 to 5, by performing the annealing step (Step 4-2), the drawn core material 30 is made to detach from the drawn composite material 10 due to the difference between the thermal expansion of the Ni-Ti-based alloy drawn material 20 and the thermal expansion of the drawn core material 30, thereby obtaining the tubing material 1. In the annealing step (Step 4-2), since the Ni-Ti-based alloy drawn material 20 uniformly expands by thermal expansion, it is possible to suppress the occurrence of rubbing, scratching, or the like due to detaching of the drawn core material 30 from the Ni-Ti-based alloy drawn material 20 on the inner wall surface of the tubular Ni-Ti-based alloy drawn material 20. In addition, by the annealing step (Step 4-2), the drawn core material 30 is detached from the drawn composite material 10 to obtain the tubing material 1, and superelastic property may be imparted to the tubing material 1.

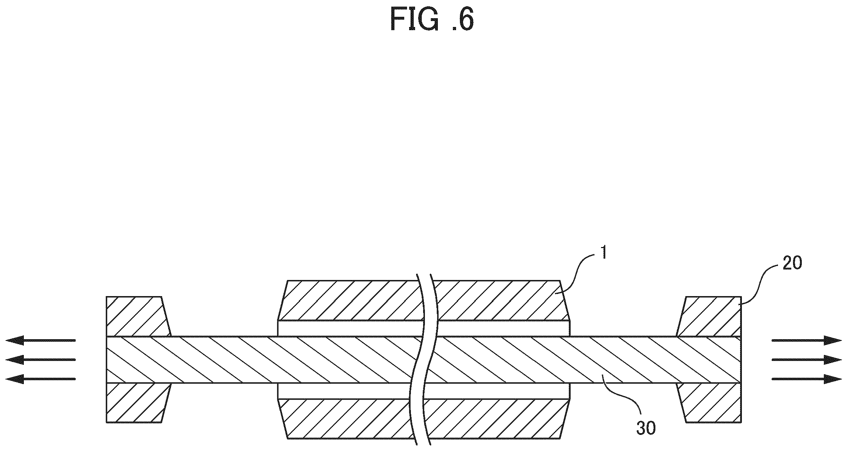

In addition, the core diameter-reducing step (Step 4-3) may be performed after the annealing step (Step 4-2). FIG. 6 is a cross-sectional view showing an example of the core diameter-reducing step (Step 4-3), and shows a state in which the core diameter-reducing step (Step 4-3) is performed on the configuration of FIG. 4. As shown in FIG. 6, in the core diameter-reducing step (Step 4-3), a tensile load is applied to the annealed drawn core material 30 separated from the tubing material 1. The tensile load applied to the drawn core material 30 in the core diameter-reducing step (Step 4-3) is larger than the tensile load applied to the drawn core material 30 in the tensile load applying step (Step 4-1). In addition, the tensile load in the core diameter-reducing step (Step 4-3) is not applied to the tubing material 1.

By applying a large tensile load to only the drawn core material 30 in the core diameter-reducing step (Step 4-3), the drawn core material 30 can be reduced in diameter. Therefore, the clearance between the inner wall surface of the tubing material 1 and the outer wall surface of the drawn core material 30 can be increased, compared to the configuration after the annealing step (Step 4-2).

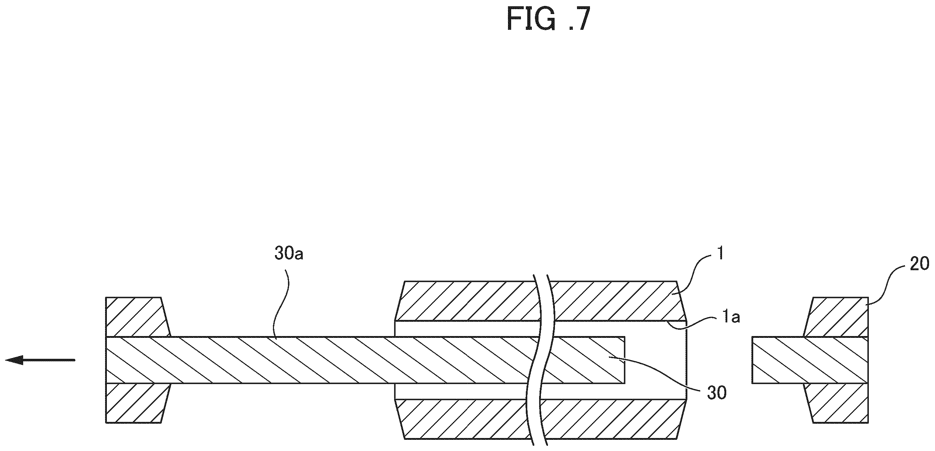

FIG. 7 is a cross-sectional view showing an example of a core extracting step (Step 4-4). As shown in FIG. 7, in the core extracting step (Step 4-4) performed after the annealing step (Step 4-2) or the core diameter-reducing step (Step 4-3), the tubing material 1 can be manufactured by extracting the drawn core material 30 from the tubing material 1. In the core extracting step (Step 4-4), since the drawn core material 30 is extracted from the tubing material 1 in a state where the inner wall surface 1a of the tubing material 1 and the outer wall surface 30a of the drawn core material 30 are completely separated from each other, it is possible to suppress the occurrence of rubbing, scratching, or the like originating from the extraction of the drawn core material 30 on the inner wall surface 1a of the tubing material 1, and thus it is possible to manufacture the tubing material 1 in which the arithmetic mean height Sa of the inner wall surface 1a is within the above range.

In addition, in the case of being the configuration shown in FIGS. 4 and 6, one end portion side of the drawn core material 30 is cut to remove one end portion of the drawn core material 30 integrated with a part of the Ni-Ti-based alloy drawn material 20, and then the drawn core material 30 is extracted from the tubing material 1, as shown in FIG. 7. Therefore, when the drawn core material 30 is extracted from the tubing material 1, since it is possible to avoid a part of the Ni-Ti-based alloy drawn material 20 attached to one end portion of the drawn core material 30 from coming into contact with the inner wall surface 1a of the tubing material 1, it is possible to prevent the occurrence of rubbing, scratching and the like on the inner wall surface 1a of the tubing material 1 originating from the Ni-Ti-based alloy drawn material 20.

In addition, when the core diameter-reducing step (Step 4-3) shown in FIG. 6 is performed, the clearance between the inner wall surface 1a of the tubing material 1 and the outer wall surface 30a of the drawn core material 30 can be increased, compared to the configuration after the annealing step (Step 4-2) shown in FIGS. 4 and 5. Therefore, in the core extracting step (Step 4-4), it is possible to further suppress the occurrence of rubbing, scratching, and the like originating from the extraction of the drawn core material 30 on the inner wall surface 1a of the tubing material 1. As a result, the arithmetic mean height Sa of the inner wall surface 1a of the tubing material 1 can be further reduced.

As described above, a small diameter tubing material having favorable roughness of the inner wall surface can be manufactured by the above-described method which is industrially easy, without performing special post-processing steps such as plug drawing processing, mandrel processing, and inner wall surface polishing, which are necessitated in the conventional technology. Further, by the above-described industrially easy method, it is possible to easily manufacture a tubing material which is longer than conventional, for example, a tubing material having a length of about 10 m to 100 m.

In addition, the manufacturing method of the tubing material according to the embodiment may further include a superelastic property imparting heat treating step (Step 5) of conducting a superelastic property imparting heating on the tubing material 1 performed after the core removing step (Step 4). The conditions for the superelastic property imparting heating such as the heating atmosphere and the heating temperature of the tubing material 1 are not particularly limited as long as capable of imparting superelastic property to the tubing material 1.

For example, when the tubing material is heated at a temperature of 600°C or lower in an inert gas atmosphere, the generated amount of non-metallic inclusions present in the tubing material consisting of the Ni-Ti-based alloy can be suppressed. In addition, the heating temperature is preferably 450°C or higher and 580°C or lower. When the superelastic property imparting heat treating step (Step 5) is performed in a state of applying the tensile load to the tubing material to stretch out straight, the straightness of the tubing material can be improved.

In addition, in the manufacturing method of the tubing material according to the embodiment, additional processing may be performed for the purpose of fine tuning the dimensions of the tubing material or for the purpose of preliminary-stage processing of the superelastic property imparting heat treating step (Step 5), at between the core removing step (Step 4) and the superelastic property imparting heat treating step (Step 5), or after the core removing step (Step 4) in the case of not performing the superelastic property imparting heat treating step (Step 5).

The total cross section reduction rate of the tubing material in the additional processing is preferably 10% or more and less than 120%, and more preferably 10% or more and less than 84%. As described in JIS H0500:1998, the total cross section reduction rate of the tubing material is a proportion of the cross-sectional area reduced by processing relative to the original cross-sectional area, and is expressed as a percentage (%) ((Ao-A)/Ao×100%) obtained by dividing the difference between the cross-sectional area Ao of the material before processing and the cross-sectional area A of the material after processing by the cross-sectional area Ao of the material before processing. Here, the cross-sectional area Ao of the tubing material before the additional processing and the cross-sectional area A of the tubing material after the additional processing are measured, and the total cross section reduction rate is calculated using the above-described calculation formula.

The tubing material of the embodiment can be industrially manufactured by an easy method as described above, the roughness of the inner wall surface is improved, and the durability against rotary bending is excellent, and hence the tubing material can be suitably used as a tubing material constituting a medical device. Suitable medical devices include stents, guide wires, and pressure guide wires. Such stents, guide wires, and pressure guide wires are each formed from a tubing material. Further, since the roughness of the inner wall surface of the tubing material is improved, a mode in which another device is inserted inside of the tubing material and used can be expected as a novel medical device.

According to the embodiment described above, it is possible to obtain, by a simple method, a tubing material which consists of a Ni-Ti-based alloy, improves the roughness of the inner wall surface, and is superior in durability against rotary bending.

Although an embodiment has been described above, the present invention is not limited to the above embodiment, and various modifications can be made within the scope of the present disclosure, including all aspects included in the gist of the present disclosure and the attached claims.

EXAMPLES

Next, Examples and Comparative Examples will be described; however, the present disclosure is not to be limited to these Examples. In addition, the composition (% by mass) of each component shown in Tables 1 and 2 was determined by the RIR (Reference Intensity Ratio) method.

(Example 1)

As a melting and casting step (Step 1), a molten metal obtained by melting Ni base metal and Ti base metal, which are Ni-Ti-based alloy materials, in a high-frequency melting furnace was poured to obtain the Ni-Ti-based alloy ingot having the alloy composition shown in Table 1. Next, as a hot working step (Step 2), the obtained ingot was forged at 500°C or higher and 950°C or lower to obtain a Ni-Ti-based alloy forged material. Subsequently, the forged material was bored with a gun drill, and then finish processed with a lathe to obtain a seamless tubular Ni-Ti-based alloy hot-worked material. Next, as a drawing step (Step 3), the drawing process was performed on a composite material obtained by inserting the core indicated in Table 3 into a tubular processed material. Thus, a drawn composite material was obtained in which the tubular Ni-Ti-based alloy drawn material and the drawn core material extending inside the Ni-Ti-based alloy drawn material are integrated.

Next, as a core removing step (Step 4), score lines were formed along the circumferential direction at both end portions of the tubular Ni-Ti-based alloy drawn material constituting the drawn composite material. Subsequently, the tensile load indicated in Table 3 was applied to the drawn core material, while grasping portions of the drawn composite material closer to the end side than the score line. Next, the Ni-Ti-based alloy drawn material was heated at the annealing temperature indicated in Table 3 in a state of applying the tensile load indicated in Table 3 to the drawn core material. Thus, superelastic property was imparted, and the drawn core material was detached from the drawn composite material to obtain a tubing material. Subsequently, one end portion side of the drawn core material was cut to remove an end portion of the drawn core material integrated with a part of the Ni-Ti-based alloy drawn material, and then the drawn core material was extracted from the tubing material. Thus, a tubing material was manufactured having a length of 10 to 100 m, and a size, an inner wall surface with the arithmetic mean height Sa and the arithmetic mean roughness Ra indicated by Table 3.

(Example 2)

A tubing material having an inner wall surface with the arithmetic mean height Sa and the arithmetic mean roughness Ra indicated in Table 3 was produced in the same manner as Example 1, except for changing the tensile load during annealing and the annealing temperature in the core removing step (Step 4) to the values indicated in Table 3, and conducting the additional processing of the total cross section reduction rate indicated in Table 3 on the tubing material.

(Examples 3 to 8)

A tubing material having an inner wall surface with the arithmetic mean height Sa and the arithmetic mean roughness Ra indicated in Table 3 was produced in the same manner as Example 1, except for using the core indicated in Table 3, and changing the tensile load during annealing and the annealing temperature in the core removing step (Step 4) to the values indicated in Table 3.

(Examples 9 to 16)

A tubing material having an inner wall surface with the arithmetic mean height Sa and the arithmetic mean roughness Ra indicated in Table 3 was produced in the same manner as Example 1, except for using the core indicated in Table 3, changing the tensile load during annealing and the annealing temperature in the core removing step (Step 4) to the values indicated in Table 3, and conducting the additional processing of the total cross section reduction rate indicated in Table 3 on the tubing material.

(Comparative Examples 1 to 4)

A tubing material having an inner wall surface with the arithmetic mean height Sa and the arithmetic mean roughness Ra indicated in Table 3 was produced in the same manner as Example 1, except for using the core indicated in Table 3, and changing the tensile load during annealing and the annealing temperature in the core removing step (Step 4) to the values indicated in Table 3. It should be noted that, in Comparative Examples 1 and 2, a tensile load was not applied during annealing, and annealing was performed in a state of not applying tensile load to the drawn core material. In addition, annealing was not performed in Comparative Example 4.

(Comparative Examples 5 to 13)

A tubing material having an inner wall surface with the arithmetic mean height Sa and the arithmetic mean roughness Ra indicated in Table 3 was produced in the same manner as Example 1, except for using the core indicated in Table 3, changing the tensile load during annealing and the annealing temperature in the core removing step (Step 4) to the values indicated in Table 3, and conducting the additional processing of the total cross section reduction rate indicated in Table 3 on the tubing material. It should be noted that, in Comparative Examples 6 to 11, a tensile load was not applied during annealing, and annealing was performed in a state of not applying tensile load to the drawn core material.

(Examples 17 to 21)

A tubing material having an inner wall surface with the arithmetic mean height Sa and the arithmetic mean roughness Ra indicated in Table 4 was produced in the same manner as Example 1, except for using the core indicated in Table 4 and repeatedly conducting the drawing process and the annealing at the temperature indicated in Table 4 on the composite material in the drawing step (Step 3), and changing the tensile load during annealing and the annealing temperature in the core removing step (Step 4) to the values indicated in Table 4.

(Examples 22 to 25)

A tubing material having an inner wall surface with the arithmetic mean height Sa and the arithmetic mean roughness Ra indicated in Table 4 was produced in the same manner as Example 1, except for using the core indicated in Table 4 and repeatedly conducting the drawing process and the annealing at the temperature indicated in Table 4 on the composite material in the drawing process (Step 3), changing the tensile load during annealing and the annealing temperature in the core removing step (Step 4) to the values indicated in Table 4, and conducting the additional processing of the total cross section reduction rate indicated in Table 4 on the tubing material.

(Examples 26 to 37)

A tubing material having the size, and an inner wall surface with the arithmetic mean height Sa and the arithmetic mean roughness Ra indicated in Table 5 was produced in the same manner as Example 1, except for using the core indicated in Table 5, changing the tensile load during annealing and the annealing temperature in the core removing step (Step 4) to the values indicated in Table 5, and conducting the additional processing of the total cross section reduction rate indicated in Table 5 on the tubing material. It should be noted that, in Examples 27 and 31, the additional processing was not performed.

(Examples 38 to 49 and Comparative Examples 14 to 15)

A tubing material having an inner wall surface with the arithmetic mean height Sa and the arithmetic mean roughness Ra indicated in Table 6 was produced in the same manner as Example 1, except for using the Ni-Ti-based alloy ingot having the alloy composition in Tables 1 and 2 as shown in Table 6, using the core indicated in Table 6, changing the tensile load during annealing and the annealing temperature in the core removing step (Step 4) to the values indicated in Table 6, and conducting the additional processing of the total cross section reduction rate indicated in Table 6 on the tubing material.

(Examples 50 to 59)

A tubing material having an inner wall surface with the arithmetic mean height Sa and the arithmetic mean roughness Ra indicated in Table 7 was produced in the same manner as Example 1, except for using the Ni-Ti-based alloy ingot having the alloy composition in Table 2 as shown in Table 7, and changing the tensile load during annealing and the annealing temperature in the core removing step (Step 4) to the values indicated in Table 7.

(Measurement and Evaluation)

The following measurements and evaluations were performed on the tubing materials obtained in the above Examples and Comparative Examples.

(1) Arithmetic mean height Sa of tubular inner wall surface

A tubular sample collected by cutting a portion of the obtained tubing material distanced 100 mm or more away from both end portions was vertically divided along the longitudinal direction, and then the inner wall surface of the tubing material was measured at four locations using a LEXT OLS5100 3D measurement laser microscope.

With 0.1<Ra ($\mu$m)<2.0, the evaluation length (mm) of the roughness curve is defined to be at least 4 mm, as described in ISO4288 1996 E (JIS B0633:2001); whereas, a detailed measurement length is not defined for the arithmetic mean height Sa. In addition, the conditions relating to three-dimensional surface properties are described in JIS B0681-2:2018 and JIS B0681-3:2019; however, in comparison with twodimensional roughness measurement, there is no definition related to the cut-offs of the L filter corresponding to $\lambda_c$ and the S filter corresponding to $\lambda_s$.

Therefore, in this measurement, the circumferential direction of the tubing material was defined as the X direction and the longitudinal direction of the tubing material was defined as the Y direction, analysis was performed using a connected image in which five fields of view are connected in the Y direction (since there are overlapping portions in the connected image, the length of the connected image in the Y direction is about 592 $\mu$m) with respect to a measurement surface (one field of view) with 35 $\mu$m in the X direction and 125 $\mu$m in the Y direction, and no cut-off value for each of the filters (L and S) was set at this time. With respect to the F operator corresponding to the boundary $\lambda$f of the undulation component defined in JIS B0681-3:2019, removal processing for the undulation component was performed using the multidimensional curve fourth-order operator, the curvature in the X and Y directions was converted into a plane, and then analysis was performed to obtain an arithmetic mean height. Then, the smallest value among the obtained arithmetic mean heights measured at the four locations was defined as the arithmetic mean height Sa of the inner wall surface of the tubing material.

(2) Arithmetic mean roughness Ra of tubular inner wall surface

In the image obtained by measuring the inner wall surface of the tubing material using a 3D measurement laser microscope in the same manner as described above, seven lines having an evaluation length of 4 mm or more along the Y direction were measured. Then, the smallest value among the arithmetic mean roughnesses measured at the four locations was defined as the arithmetic mean roughness Ra of the inner wall surface of the tubing material.

(3) Arithmetic mean height Sa of outer wall surface of core

The outer wall surface of the core was measured using a 3D measurement laser microscope in the same manner as described above on the portions of the core distanced 100 mm or more away from both end portions and on the central portion of the core, and the arithmetic mean height was obtained. Then, among the arithmetic mean heights obtained at the measured three locations, a numerical value obtained by summarizing the value of the second decimal place of the largest value for every 0.05 $\mu$m as follows was set as the arithmetic mean height Sa of the core outer wall surface. Specifically, a value of 0.08 $\mu$m or more and 0.10 $\mu$m or less was set as an arithmetic mean height Sa of 0.10 $\mu$m, a value of 0.11 $\mu$m or more and 0.15 $\mu$m or less was set as an arithmetic mean height Sa of 0.15 $\mu$m, a value of 0.16 $\mu$m or more and 0.20 $\mu$m or less was set as an arithmetic mean height Sa of 0.20 $\mu$m, and a value of 0.25 $\mu$m or more and 0.30 $\mu$m or less was set as an arithmetic mean height Sa of 0.30 $\mu$m. It should be noted that, since the 0.05 $\mu$m of Example 18 and the 0.03 $\mu$m of Example 19 in Table 1 were values too small to be summarized by 0.05 according to the above, the largest value was defined as the arithmetic mean height Sa of the core outer wall surface.

(4) Fracture resistance to bending strain of 1.0%

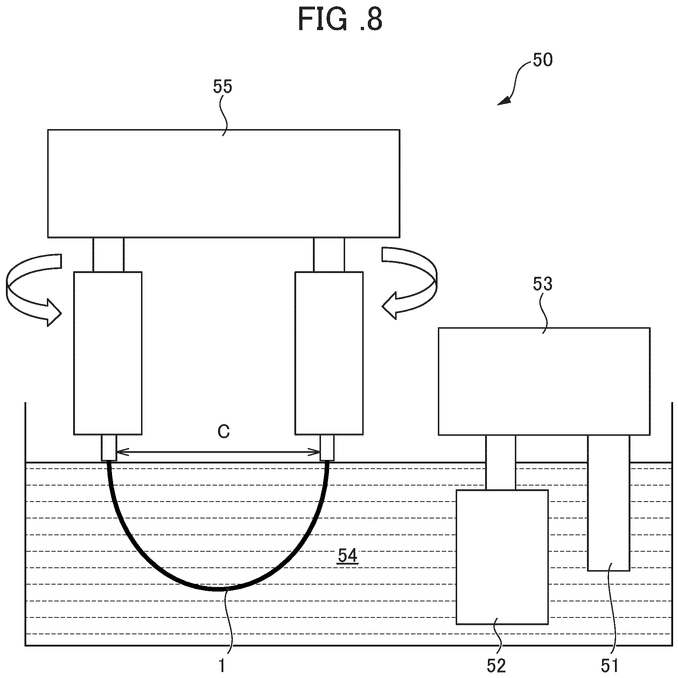

As the durability against rotary bending, a rotary bending test was performed in a state where a bending strain of 1.0% was applied to the tubing material, and the fracture resistance against the strain was measured. Specifically, using the dual-drive type rotary bending fatigue tester 50 shown in FIG. 8, a fracture resistance test was performed on the tubing material 1 against a bending strain of 1.0%. It should be noted that the maximum bending strain 1.0% (strain) applied to the tubing material was set according to the following Formula (1). The chuck-to-chuck distance C was set according to the following Formula (2). R is the outer diameter of the tubing material, and L is the length of the tubing material.

With the dual-drive type rotary bending fatigue tester 50, the temperature of silicone oil 54 was maintained at 37°C, while the silicone oil 54 was circulated by a stirrer motorequipped heater power supply 53 including a heater 51 and a stirring device 52. Then, the tubing material 1 was rotated to a rotation number of 300 rotations/minute in a state where a bending strain of 1.0% was applied to the tubing material 1 by a revolution counter-equipped motor 55, and the count until the tubing material 1 was fractured (fracture count) was measured. However, only in Example 49 in which the transformation temperature was high, the temperature of the silicone oil 54 was maintained at 100°C. This measurement was performed on three samples (n=3), and the lowest count was defined as the fracture count. The fracture resistance was ranked as follows.

⊙ : fracture count was 9000 or more.
O : fracture count was 7500 or more and less than 9000.
x : fracture count was less than 7500.

[Formula 1]

$$C = \frac{1.198 \times R}{strain} \qquad (1)$$

[Formula 2]

$$L = 2.19 \times C \qquad (2)$$

It should be noted that, since the rotary bending test is affected by the physical state (roughness, voids, defects, etc.) of the sample surface, the physical state of the sample surface is generally made constant by subjecting the sample surface to electrolytic polishing. However, in the case of high strain bending of the tubing material, it has become evident that the electropolished state of the tubing material affects the fracture count of the tubing material; therefore, herein, the rotary bending test was performed after processing the arithmetic mean height Sa of the outer wall surface of the tubing material to 0.15 $\mu$m or more and 0.25 $\mu$m or less.

(5) Property evaluation of tubing material

With respect to the tubing materials produced in Examples 1 to 59, since the residual strain was 0.5% or less by unloading after applying a strain of 6% in the tensile test, or by heating after the unloading, the tubing materials of Examples 1 to 59 had superelastic property and shape memory property.

(6) Oxygen concentration and carbon concentration

The oxygen concentration and the carbon concentration contained in the tubing materials produced in Examples 1 to 59 were measured using a carbon concentration analyzer and an oxygen concentration analyzer. As a result, both were 0.04% by mass or less.

[Table 1]

| Alloy No. | Composition (% by mass) | |
| --- | --- | --- |
| | Ni | Balance |
| A | 55.8 | Ti + inevitable impurities |
| B | 56.0 | Ti + inevitable impurities |
| C | 56.1 | Ti + inevitable impurities |
| D | 56.0 | Ti + inevitable impurities |
| E | 55.9 | Ti + inevitable impurities |
| F | 55.8 | Ti + inevitable impurities |
| G | 56.0 | Ti + inevitable impurities |
| H | 56.2 | Ti + inevitable impurities |

(continued)

| Alloy No. | Composition (% by mass) | |
|---|---|---|
| | Ni | Balance |
| I | 56.7 | Ti + inevitable impurities |
| J | 56.1 | Ti + inevitable impurities |
| K | 55.6 | Ti + inevitable impurities |
| L | 55.0 | Ti + inevitable impurities |
| a | 54.3 | Ti + inevitable impurities |
| b | 57.1 | Ti + inevitable impurities |

[Table 2]

| Alloy No. | Composition (% by mass) | | | | | | | | | | |
|---|---|---|---|---|---|---|---|---|---|---|---|
| | Ni | Cu | Ta | Zr | Nb | v | Mo | Cr | Fe | Co | Balance |
| M | 56.2 | 0.05 | - | - | - | - | - | - | - | - | Ti + inevitable impurities |
| N | 56.1 | - | 0.05 | - | - | - | - | - | - | - | Ti + inevitable impurities |
| O | 56.1 | - | - | 0.05 | - | - | - | - | - | - | Ti + inevitable impurities |
| P | 56.2 | - | - | - | 0.05 | - | - | - | - | - | Ti + inevitable impurities |
| Q | 56.1 | - | - | - | - | 0.05 | - | - | - | - | Ti + inevitable impurities |
| R | 56.1 | - | - | - | - | - | 0.05 | - | - | - | Ti + inevitable impurities |
| S | 56.1 | - | - | - | - | - | - | 0.05 | - | - | Ti + inevitable impurities |
| T | 56.2 | - | - | - | - | - | - | - | 0.05 | - | Ti + inevitable impurities |
| U | 56.1 | - | - | - | - | - | - | - | - | 0.05 | Ti + inevitable impurities |
| V | 56.1 | 0.01 | - | - | - | - | - | 0.02 | - | - | Ti + inevitable impurities |

[Table 3]

| | Alloy No. | Tube size | | | Drawing step (Step 3) | Core removing step (Step 4) | | Additional processing | Inner wall surface | | Fracture resistance |
| | | Outer diameter R | Inner diameter r | Ratio (R/r) | Core material | Tensile load during annealing | Annealing temperature | Total cross section reduction rate | Ra | Sa | |
| | | (mm) | (mm) | - | | (MPa) | (°C) | (%) | (μm) | (μm) | |
| Example 1 | A | 0.50 | 0.30 | 1.67 | SUS304 | 100 | 500 | - | 0.26 | 0.31 | ◎ |
| Example 2 | A | 0.50 | 0.30 | 1.67 | SUS304 | 20 | 700 | 45 | 0.32 | 0.40 | ◎ |
| Example 3 | A | 0.50 | 0.30 | 1.67 | SUJ2 | 150 | 450 | - | 0.29 | 0.33 | ◎ |
| Example 4 | A | 0.50 | 0.30 | 1.67 | SS400 | 150 | 450 | - | 0.28 | 0.35 | ◎ |
| Example 5 | A | 0.50 | 0.30 | 1.67 | SCM415 | 40 | 500 | - | 0.32 | 0.34 | ◎ |
| Example 6 | A | 0.50 | 0.30 | 1.67 | SUS316 | 40 | 500 | - | 0.30 | 0.39 | ◎ |
| Example 7 | A | 0.50 | 0.30 | 1.67 | SUS309S | 40 | 550 | - | 0.31 | 0.34 | ◎ |
| Example 8 | A | 0.50 | 0.30 | 1.67 | SUS310S | 20 | 550 | - | 0.27 | 0.33 | ◎ |
| Example 9 | A | 0.50 | 0.30 | 1.67 | 55.1%Ni-Ti+ balance | 150 | 700 | 60 | 0.41 | 0.45 | ◎ |
| Example 10 | A | 0.50 | 0.30 | 1.67 | 55.1%Ni-Ti+ balance | 20 | 700 | 60 | 0.41 | 0.47 | ○ |
| Example 11 | A | 0.50 | 0.30 | 1.67 | 54.5%Ni-Ti+ balance | 150 | 700 | 60 | 0.42 | 0.48 | ○ |
| Example 12 | A | 0.50 | 0.30 | 1.67 | 56.0%Ni-Ti+ balance | 150 | 700 | 60 | 0.40 | 0.48 | ○ |
| Example 13 | A | 0.50 | 0.30 | 1.67 | SUJ2 | 8 | 700 | 55 | 0.39 | 0.49 | ○ |
| Example 14 | A | 0.50 | 0.30 | 1.67 | 55.1%Ni-Ti+ balance | 300 | 700 | 80 | 0.42 | 0.53 | ○ |
| Example 15 | A | 0.50 | 0.30 | 1.67 | SCM415 | 40 | 800 | 10 | 0.30 | 0.33 | ○ |
| Example 16 | A | 0.50 | 0.30 | 1.67 | SCM415 | 40 | 150 | 10 | 0.31 | 0.34 | ○ |
| Comparative Example 1 | A | 0.50 | 0.30 | 1.67 | SUJ2 | None | 500 | - | - | - | - |

EP 4 556 588 A1

| | Alloy No. | Tube size | | | Drawing step (Step 3) | Core removing step (Step 4) | | Additional processing | Inner wall surface | | Fracture resistance |
| | | Outer diameter R | Inner diameter r | Ratio (R/r) | Core material | Tensile load during annealing | Annealing temperature | Total cross section reduction rate | Ra | Sa | |
| | | (mm) | (mm) | - | | (MPa) | (°C) | (%) | (μm) | (μm) | |
| Comparative Example 2 | A | 0.50 | 0.30 | 1.67 | SS400 | None | 450 | - | - | - | - |
| Comparative Example 3 | A | 0.50 | 0.30 | 1.67 | SCM415 | 450 | 450 | - | - | - | - |
| Comparative Example 4 | A | 0.50 | 0.30 | 1.67 | SUS310S | 40 | None | - | - | - | - |
| Comparative Example 5 | A | 0.50 | 0.30 | 1.67 | 55.1%Ni-Ti+ balance | 40 | 1000 | 45 | 0.55 | 0.58 | × |
| Comparative Example 6 | A | 0.50 | 0.30 | 1.67 | 55.1%Ni-Ti+ balance | None | 700 | 45 | 0.55 | 0.63 | × |
| Comparative Example 7 | A | 0.50 | 0.30 | 1.67 | 54.5%Ni-Ti+ balance | None | 600 | 45 | 0.56 | 0.81 | × |
| Comparative Example 8 | A | 0.50 | 0.30 | 1.67 | 56.0%Ni-Ti+ balance | None | 600 | 45 | 0.60 | 0.85 | × |
| Comparative Example 9 | A | 0.50 | 0.30 | 1.67 | SUS310S | None | 750 | 45 | 0.51 | 0.59 | × |
| Comparative Example 10 | A | 0.50 | 0.30 | 1.67 | 54.5%Ni-Ti+ balance | None | 600 | 45 | 0.59 | 0.75 | × |
| Comparative Example 11 | A | 0.50 | 0.30 | 1.67 | 56.0%Ni-Ti+ balance | None | 600 | 45 | 0.59 | 0.70 | × |
| Comparative Example 12 | A | 0.50 | 0.30 | 1.67 | SUS304 | 40 | 500 | 130 | 0.79 | 120 | × |
| Comparative Example 13 | A | 0.50 | 0.30 | 1.67 | 55.1%Ni-Ti+ balance | 40 | 700 | 120 | 0.78 | 1.00 | × |

16

[Table 4]

| | Alloy No. | Tube size | | | Drawing step (Step 3) | | | Core removing step (Step 4) | | Additional processing | Inner wall surface | | Fracture resistance |
| | | Outer diameter R | Inner diameter r | Ratio (R/r) | | | Annealing | | | | | | |
| | | | | | Core | | | | | | | | |
| | | | | | Material | Outer wall surface Sa | Temperature | Tensile load during annealing | Annealing temperature | Total cross section reduction rate | Ra | Sa | |
| | | (mm) | (mm) | - | | (μm) | (°C) | (MPa) | (°C) | (%) | (μm) | (μm) | (count) |
| Example 17 | A | 0.50 | 0.30 | 1.67 | SUS304 | 0.30 | 700 | 150 | 500 | - | 0.41 | 0.50 | ○ |
| Example 18 | A | 0.50 | 0.30 | 1.67 | SUS304 | 0.05 | 700 | 150 | 500 | - | 0.06 | 0.08 | ◎ |
| Example 19 | A | 0.50 | 0.30 | 1.67 | SUS304 | 0.03 | 700 | 150 | 500 | - | 0.06 | 0.08 | ◎ |
| Example 20 | A | 0.50 | 0.30 | 1.67 | SS400 | 0.10 | 800 | 40 | 450 | - | 0.31 | 0.32 | ◎ |
| Example 21 | A | 0.50 | 0.30 | 1.67 | SS400 | 0.10 | 500 | 40 | 500 | - | 0.30 | 0.34 | ◎ |
| Example 22 | A | 0.50 | 0.30 | 1.67 | SUJ2 | 0.10 | 700 | 4 | 700 | 55 | 0.39 | 0.49 | ○ |
| Example 23 | A | 0.50 | 0.30 | 1.67 | 55.1%Ni-Ti + balance | 0.10 | 700 | 300 | 700 | 80 | 0.40 | 0.53 | ○ |
| Example 24 | A | 0.50 | 0.30 | 1.67 | SCM415 | 0.15 | 700 | 100 | 800 | 10 | 0.29 | 0.33 | ○ |
| Example 25 | A | 0.50 | 0.30 | 1.67 | SCM415 | 0.15 | 700 | 100 | 150 | 10 | 0.31 | 0.34 | ○ |

17

[Table 5]

| | Alloy No. | Tube size | | | Drawing step (Step 3) | Core removing step (Step 4) | | Additional processing | Inner wall surface | | Fracture resistance |
|---|---|---|---|---|---|---|---|---|---|---|---|
| | | Outer diameter R | Inner diameter r | Ratio (R/r) | Core material | Tensile load during annealing | Annealing temperature | Total cross section reduction rate | Ra | Sa | |
| | | (mm) | (mm) | - | | (MPa) | (°C) | (%) | (μm) | (μm) | |
| Example 26 | A | 4.00 | 1.60 | 2.50 | SUS304 | 40 | 750 | 42 | 0.33 | 0.39 | ◎ |
| Example 27 | A | 4.00 | 3.95 | 1.01 | 55.1%Ni-Ti + balance | 200 | 500 | - | 0.31 | 0.34 | ◎ |
| Example 28 | A | 2.00 | 1.55 | 1.29 | 55.1%Ni-Ti + balance | 200 | 650 | 20 | 0.28 | 0.31 | ◎ |
| Example 29 | A | 3.50 | 3.10 | 1.13 | SUJ2 | 80 | 700 | 35 | 0.28 | 0.38 | ◎ |
| Example 30 | A | 0.75 | 0.55 | 1.36 | SUJ2 | 80 | 700 | 57 | 0.40 | 0.44 | ◎ |
| Example 31 | A | 3.11 | 2.14 | 1.45 | SS400 | 100 | 500 | - | 0.26 | 0.30 | ◎ |
| Example 32 | A | 1.00 | 0.50 | 2.00 | SS400 | 100 | 700 | 38 | 0.31 | 0.35 | ◎ |
| Example 33 | A | 1.00 | 0.75 | 1.33 | 55.1%Ni-Ti + balance | 200 | 650 | 35 | 0.33 | 0.35 | ◎ |
| Example 34 | A | 0.50 | 0.40 | 1.25 | 55.1%Ni-Ti + balance | 200 | 650 | 60 | 0.39 | 0.45 | ◎ |
| Example 35 | A | 0.30 | 0.12 | 2.50 | SUJ2 | 40 | 750 | 83 | 0.45 | 0.54 | ○ |
| Example 36 | A | 0.15 | 0.10 | 1.50 | SUJ2 | 40 | 750 | 55 | 0.40 | 0.48 | ○ |
| Example 37 | A | 0.50 | 0.10 | 5.00 | SUS304 | 40 | 750 | 10 | 0.25 | 0.29 | ◎ |

EP 4 556 588 A1

[Table 6]

| | Alloy No. | Tube size | | | Drawing step (Step 3) | Core removing step (Step 4) | | Additional processing | Inner wall surface | | Fracture resistance |
|---|---|---|---|---|---|---|---|---|---|---|---|
| | | Outer diameter R | Inner diameter r | Ratio (R/r) | Core material | Tensile load during annealing | Annealing temperature | Total cross section reduction rate | Ra | Sa | |
| | | (mm) | (mm) | - | | (MPa) | (°C) | (%) | (μm) | (μm) | |
| Example 38 | A | 0.50 | 0.30 | 1.67 | SUS304 | 40 | 700 | 35 | 0.33 | 0.38 | ◎ |
| Example 39 | B | 0.50 | 0.30 | 1.67 | SUS304 | 40 | 700 | 35 | 0.31 | 0.39 | ◎ |
| Example 40 | C | 0.50 | 0.30 | 1.67 | SUS304 | 40 | 700 | 35 | 0.32 | 0.39 | ◎ |
| Example 41 | D | 0.50 | 0.30 | 1.67 | SCM415 | 40 | 700 | 35 | 0.27 | 0.38 | ◎ |
| Example 42 | E | 0.50 | 0.30 | 1.67 | SUS304 | 40 | 700 | 35 | 0.30 | 0.40 | ◎ |
| Example 43 | F | 0.50 | 0.30 | 1.67 | 55.1%Ni-Ti + balance | 40 | 700 | 35 | 0.30 | 0.39 | ◎ |
| Example 44 | G | 0.50 | 0.30 | 1.67 | SS400 | 40 | 700 | 35 | 0.31 | 0.41 | ◎ |
| Example 45 | H | 0.50 | 0.30 | 1.67 | SUS304 | 40 | 700 | 35 | 0.32 | 0.40 | ◎ |
| Example 46 | I | 0.50 | 0.30 | 1.67 | SUS304 | 40 | 700 | 35 | 0.28 | 0.41 | ◎ |
| Example 47 | J | 0.50 | 0.30 | 1.67 | SUJ2 | 40 | 700 | 35 | 0.29 | 0.40 | ◎ |
| Example 48 | K | 0.50 | 0.30 | 1.67 | SUS304 | 40 | 700 | 35 | 0.33 | 0.41 | ○ |
| Example 49 | M | 0.50 | 0.30 | 1.67 | SUS304 | 40 | 700 | 35 | 0.34 | 0.39 | ○ |
| Comparative Example 14 | a | - | - | - | - | - | - | - | - | - | - |
| Comparative Example 15 | b | - | - | - | - | - | - | - | - | - | - |

19

[Table 7]

| | Alloy No. | Tube size | | | Drawing step (Step 3) | Core removing step (Step 4) | | Additional processing | Inner wall surface | | Fracture resistance |
|---|---|---|---|---|---|---|---|---|---|---|---|
| | | Outer diameter R | Inner diameter r | Ratio (R/r) | Core material | Tensile load during annealing | Annealing temperature | Total cross section reduction rate | Ra | Sa | |
| | | (mm) | (mm) | - | | (MPa) | (°C) | (%) | (μm) | (μm) | |
| Example 50 | M | 0.50 | 0.30 | 1.67 | SUS304 | 100 | 500 | - | 0.32 | 0.34 | ◎ |
| Example 51 | N | 0.50 | 0.30 | 1.67 | SUS304 | 100 | 500 | - | 0.32 | 0.36 | ◎ |
| Example 52 | O | 0.50 | 0.30 | 1.67 | SUS304 | 100 | 500 | - | 0.30 | 0.37 | ◎ |
| Example 53 | P | 0.50 | 0.30 | 1.67 | SUS304 | 100 | 500 | - | 0.28 | 0.37 | ◎ |
| Example 54 | Q | 0.50 | 0.30 | 1.67 | SUS304 | 100 | 500 | - | 0.32 | 0.35 | ◎ |
| Example 55 | R | 0.50 | 0.30 | 1.67 | SUS304 | 100 | 500 | - | 0.30 | 0.34 | ◎ |
| Example 56 | S | 0.50 | 0.30 | 1.67 | SUS304 | 100 | 500 | - | 0.31 | 0.38 | ◎ |
| Example 57 | T | 0.50 | 0.30 | 1.67 | SUS304 | 100 | 500 | - | 0.27 | 0.37 | ◎ |
| Example 58 | U | 0.50 | 0.30 | 1.67 | SUS304 | 100 | 500 | - | 0.33 | 0.38 | ◎ |
| Example 59 | v | 0.50 | 0.30 | 1.67 | SUS304 | 100 | 500 | - | 0.32 | 0.38 | ◎ |

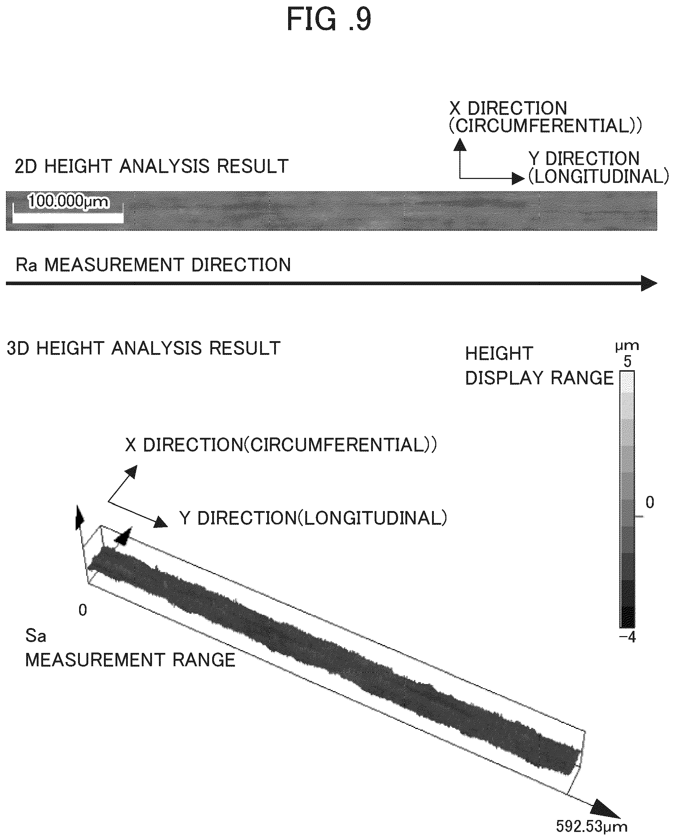

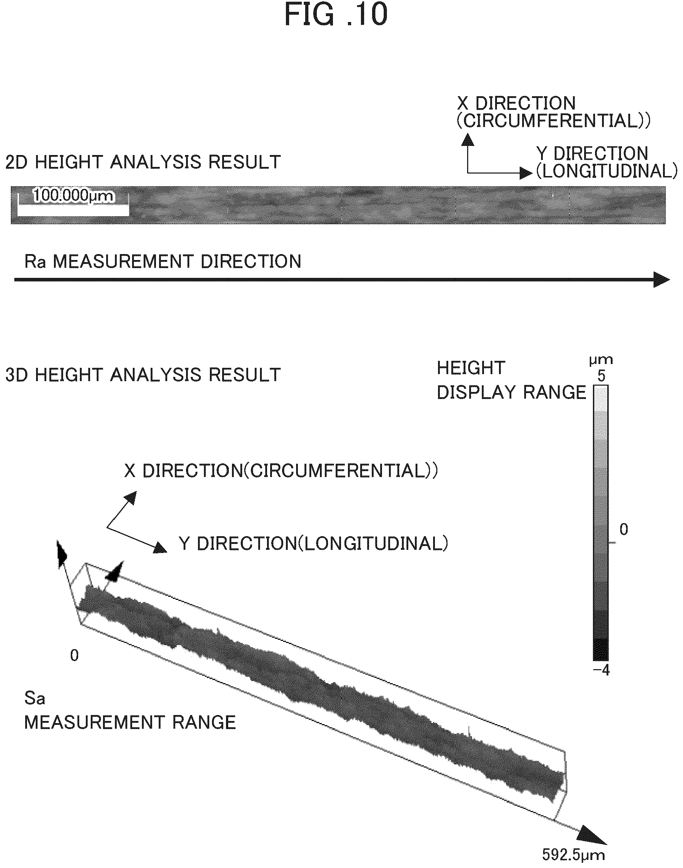

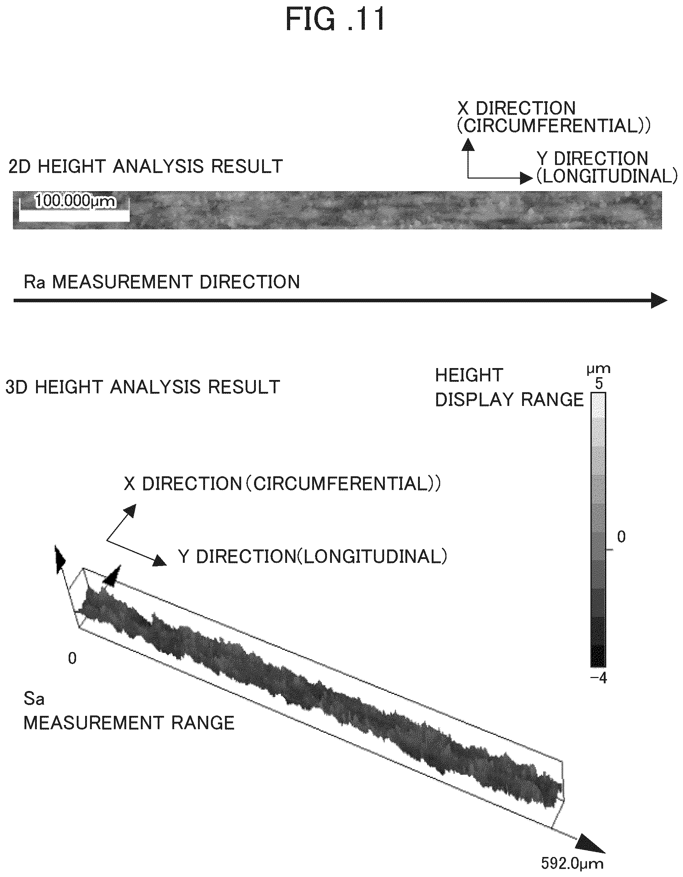

FIG. 9 shows the results of observing the inner wall surface of the tubing material produced in Example 1 by a 3D measurement laser microscope. FIG. 10 shows the results of observing the inner wall surface of the tubing material produced in Example 10 by a 3D measurement laser microscope. FIG. 11 shows the results of observing the inner wall surface of the tubing material produced in Comparative Example 6 by a 3D measurement laser microscope.

As shown in Tables 1 to 7 and FIGS. 9 to 11, in all the Examples, the tubing materials produced by the process under the predetermined conditions consisted of a Ni-Ti-based alloy, and the arithmetic mean height Sa of the inner wall surface was 0.08 μm or more and 0.55 μm or less. Therefore, the durability against rotary bending was excellent. On the other hand, in all of the Comparative Examples, since the arithmetic mean height Sa of the inner wall surface was more than 0.55 μm in the tubing material produced by the process deviating from the predetermined conditions, the durability against rotary bending was poor.

Specifically, based on the results of Examples 1 to 16, it was possible to control the arithmetic mean height Sa of the inner wall surface to within the above range since the tubing material was produced by the process under the predetermined conditions, even when the material of the core was changed. Based on the results of Examples 17 to 25, it was possible to control the arithmetic mean height Sa of the inner wall surface to within the above range, even when Sa of the outer wall surface of the core was changed in the drawing step (Step 3). In addition, the arithmetic mean height Sa of the inner wall surface could be controlled to within the above range, even when drawing and annealing were repeatedly performed in the drawing step (Step 3). Based on the results of Examples 26 to 37, it was possible to control the arithmetic mean height Sa of the inner wall surface to within the above range, for the tubing materials having different outer diameters and inner diameters. Based on the results of Examples 38 to 59, it was possible to control the arithmetic mean height Sa of the inner wall surface to within the above range, for the tubing materials having different alloy compositions.

In addition, in Comparative Examples 1 and 2, since tensile load was not applied during the annealing, and the annealing was performed in a state where the tensile load was not applied to the drawn core material, the drawn core material could not be detached from the drawn composite material, and as a result, the tubing material could not be manufactured. In Comparative Example 3, for the drawn composite material, since a tensile load of a magnitude at which the tubular Ni-Ti-based alloy drawn material detaches from the drawn core material was applied to the drawn core material, the Ni-Ti-based alloy drawn material was plastically deformed, and the tube shape could not be controlled; therefore, it was determined as impossible to manufacture the tubing material. In Comparative Example 4, since annealing was not performed in a state of applying a tensile load to the drawn core material, the drawn core material could not be detached from the drawn composite material, and as a result, the tubing material could not be manufactured. In Comparative Example 5, since the annealing temperature was more than 800°C, control of the non-metallic inclusions was insufficient; therefore, the Sa of the inner wall surface of the tubing material was more than 0.55 μm, and the durability against rotary bending was inferior to that of the tubing material of Examples. In Comparative Examples 6 to 11, depending on the material of the core, a tubing material could be manufactured, even when a tensile load was not applied during annealing, and annealing was performed in a state of not applying a tensile load to the drawn core material. However, when the drawn core material was extracted from the tubing material, frictional drag which did not occur in the Examples was felt. As a result, the Sa of the inner wall surface of the tubing material was more than 0.55 μm, and the durability against rotary bending was inferior to that of the tubing materials of the Examples. In Comparative Examples 12 and 13, since the total cross section reduction rate of the additional processing on the tubing material was too large, the Sa of the inner wall surface of the tubing material was more than 0.55 μm, and the durability against rotary bending was inferior to that of the tubing materials of the Examples. In Comparative Example 14, since the Ni concentration of the alloy composition was low, cracking occurred from forging; therefore, the subsequent steps could not be performed. In Comparative Example 15, since the Ni concentration of the alloy composition was high, cracking occurred similarly to Comparative Example 14; therefore, the subsequent steps could not be performed.

EXPLANATION OF REFERENCE NUMERALS

1 tubing material
1a inner wall surface of tubing material
10 drawn composite material
20 tubular Ni-Ti-based alloy drawn material
21 score line
30 drawn core material
30a outer wall surface of drawn core material
50 dual-drive type rotary bending fatigue tester
51 heater
52 stirring device

53 stirrer motor-equipped heater power supply
54 silicone oil
55 revolution counter-equipped motor

**Claims**

1. A tubing material consisting of a Ni-Ti-based alloy, wherein an arithmetic mean height Sa of an inner wall surface is 0.08 $\mu$m or more and 0.55 $\mu$m or less.

2. The tubing material according to claim 1, wherein the tubing material contains 54.5% by mass or more and 57.0% by mass or less of Ni, and a balance consists of Ti and inevitable impurities.

3. The tubing material according to claim 1, wherein a ratio (outer diameter/inner diameter) of an outer diameter to an inner diameter of the tubing material is 1.01 or more and 5.00 or less.

4. The tubing material according to claim 1, wherein an outer diameter of the tubing material is 0.15 mm or more and 4.00 mm or less, and an inner diameter of the tubing material is 0.10 mm or more and 3.95 mm or less.

5. The tubing material according to claim 1, wherein the tubing material has superelastic property at 37°C.

6. A stent formed from the tubing material according to any one of claims 1 to 5.

7. A guide wire formed from the tubing material according to any one of claims 1 to 5.

8. A pressure guide wire formed from the tubing material according to any one of claims 1 to 5.

9. A method for producing the tubing material according to any one of claims 1 to 5, the method comprising:

   a melting and casting step for a Ni-Ti-based alloy material (Step 1);
   a hot working step (Step 2);
   a drawing step of drawing a composite material obtained by inserting a core into the tubular Ni-Ti-based alloy hot-worked material obtained in the hot working step (Step 2) to obtain a drawn composite material in which a tubular Ni-Ti-based alloy drawn material and a drawn core material extending inside of the Ni-Ti-based alloy drawn material are integrated (Step 3); and
   a core removing step of heating the Ni-Ti-based alloy drawn material at 150°C or higher and 800°C or lower, while applying, to the drawn core material in the drawn composite material, a tensile load of a magnitude at which the Ni-Ti-based alloy drawn material in the drawn composite material does not detach from the drawn core material (Step 4).

10. The method for producing the tubing material according to claim 9, wherein, in the core removing step (Step 4), the Ni-Ti-based alloy drawn material is heated at 150°C or higher and 800°C or lower, while applying a tensile load of 4 MPa or more and 300 MPa or less to the drawn core material in the drawn composite material.

11. The method for producing the tubing material according to claim 9, further comprising a superelastic property imparting heat treating step of conducting a superelastic property imparting heating on the tubing material (Step 5) performed after the core removing step (Step 4).

# FIG .1

# FIG .2

# FIG .3

# FIG .4

# FIG .5

FIG .6

FIG .7

# FIG .8

# FIG .9

2D HEIGHT ANALYSIS RESULT

X DIRECTION
(CIRCUMFERENTIAL))

Y DIRECTION
(LONGITUDINAL)

100.000μm

Ra MEASUREMENT DIRECTION

3D HEIGHT ANALYSIS RESULT

HEIGHT
DISPLAY RANGE

μm
5

0

−4

X DIRECTION(CIRCUMFERENTIAL))

Y DIRECTION(LONGITUDINAL)

0

Sa
MEASUREMENT RANGE

592.53μm

# FIG .10

2D HEIGHT ANALYSIS RESULT

X DIRECTION
(CIRCUMFERENTIAL))

Y DIRECTION
(LONGITUDINAL)

100.000μm

Ra MEASUREMENT DIRECTION

3D HEIGHT ANALYSIS RESULT

HEIGHT
DISPLAY RANGE

μm
5

0

−4

X DIRECTION(CIRCUMFERENTIAL))

Y DIRECTION(LONGITUDINAL)

0

Sa
MEASUREMENT RANGE

592.5μm

# FIG .11

2D HEIGHT ANALYSIS RESULT

X DIRECTION
(CIRCUMFERENTIAL))

Y DIRECTION
(LONGITUDINAL)

100.000μm

Ra MEASUREMENT DIRECTION

3D HEIGHT ANALYSIS RESULT

HEIGHT
DISPLAY RANGE

μm
5

0

−4

X DIRECTION(CIRCUMFERENTIAL))

Y DIRECTION(LONGITUDINAL)

0

Sa
MEASUREMENT RANGE

592.0μm

## INTERNATIONAL SEARCH REPORT

| International application No. |
| --- |
| **PCT/JP2024/011547** |

**A.    CLASSIFICATION OF SUBJECT MATTER**

*C22C 19/03*(2006.01)i; *C22F 1/00*(2006.01)i; *C22F 1/10*(2006.01)i
FI:    C22C19/03 Z; C22F1/10 Z; C22F1/00 626; C22F1/00 627; C22F1/00 630G; C22F1/00 630Z; C22F1/00 625; C22F1/00 681; C22F1/00 682; C22F1/00 683; C22F1/00 691B; C22F1/00 691Z; C22F1/00 694Z; C22F1/00 694A

According to International Patent Classification (IPC) or to both national classification and IPC

**B.    FIELDS SEARCHED**

Minimum documentation searched (classification system followed by classification symbols)

C22C19/03; C22F1/00; C22F1/10

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
| --- | --- | --- |
| A | JP 2011-206392 A (TERUMO KABUSHIKI KAISHA) 20 October 2011 (2011-10-20) paragraphs [0026]-[0032], [0039] | 1-11 |
| A | JP 2016-27200 A (TOHOKU UNIVERSITY) 18 February 2016 (2016-02-18) | 1-11 |
| A | JP 2001-96307 A (TOKIN CORP.) 10 April 2001 (2001-04-10) | 1-11 |
| A | WO 2020/039658 A1 (JAROC KK) 27 February 2020 (2020-02-27) | 1-11 |
| A | JP 1-122608 A (NKK CORP.) 15 May 1989 (1989-05-15) | 1-11 |
| A | CN 114850219 A (XI'AN NUOBOER RARE METAL MATERIALS CO., LTD.) 05 August 2022 (2022-08-05) | 1-11 |
| A | JP 2005-502472 A (MEMRY CORP.) 27 January 2005 (2005-01-27) | 1-11 |
| A | CN 115228955 A (BEIHANG UNIVERSITY) 25 October 2022 (2022-10-25) | 1-11 |

☐ Further documents are listed in the continuation of Box C.        ☑ See patent family annex.

| | |
| --- | --- |
| *    Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
| --- | --- |
| **28 May 2024** | **04 June 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT
### Information on patent family members

International application No.

**PCT/JP2024/011547**

| Patent document cited in search report | | | Publication date (day/month/year) | Patent family member(s) | | | Publication date (day/month/year) |
|---|---|---|---|---|---|---|---|
| JP | 2011-206392 | A | 20 October 2011 | (Family: none) | | | |
| JP | 2016-27200 | A | 18 February 2016 | (Family: none) | | | |
| JP | 2001-96307 | A | 10 April 2001 | (Family: none) | | | |
| WO | 2020/039658 | A1 | 27 February 2020 | JP | 2020-32459 | A | |
| JP | 1-122608 | A | 15 May 1989 | (Family: none) | | | |
| CN | 114850219 | A | 05 August 2022 | (Family: none) | | | |
| JP | 2005-502472 | A | 27 January 2005 | US | 2003/0110825 | A1 | |
| | | | | WO | 2003/024639 | A1 | |
| | | | | EP | 1427550 | A1 | |
| | | | | DE | 60224290 | T2 | |
| | | | | CA | 2460064 | A1 | |
| | | | | CN | 1555298 | A | |
| CN | 115228955 | A | 25 October 2022 | (Family: none) | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H1017963 A
- JP H1161301 A
- US 5709021 A
- US 6799357 B
- JP 3443206 B
- JP 2133478 A

**Non-patent literature cited in the description**

- *J. Materials processing Technology*, 2001, vol. 118, 251-255
- *J. Materials processing Technology*, 2004, vol. 153-154, 145-150